Europäisches Patentamt

European Patent Office

Office européen des brevets

(11) Numéro de publication: **0 395 523**
**A1**

(12) **DEMANDE DE BREVET EUROPEEN**

(21) Numéro de dépôt: 90401142.6

(51) Int. Cl.5: **A61B 6/00, G01T 1/29**

(22) Date de dépôt: 26.04.90

(30) Priorité: 28.04.89 FR 8905665

(43) Date de publication de la demande:
**31.10.90 Bulletin 90/44**

(84) Etats contractants désignés:
**DE GB NL**

(71) Demandeur: **GENERAL ELECTRIC CGR S.A.**
**100, rue Camille-Desmoulins**
**F-92130 Issy les Moulineaux(FR)**

(72) Inventeur: **Tirelli, Marco**

Cabinet Ballot-Schmit, 7 rue le Sueur
F-75116 Paris(FR)
Inventeur: **Romeas, René**
Cabinet Ballot-Schmit, 7 rue le Sueur
F-75116 Paris(FR)
Inventeur: **Gregoire, Yves**
Cabinet Ballot-Schmit, 7 rue le Sueur
F-75116 Paris(FR)

(74) Mandataire: **Ballot, Paul Denis Jacques et al**
**Cabinet Ballot-Schmit 7, rue le Sueur**
**F-75116 Paris(FR)**

(54) **Dispositif de contrôle de radiation à surface active modulable du type sensible au rayonnement ionisant.**

(57) L'invention concerne un système de radiologie du type mammographe dans lequel il est prévu un contrôleur de radiation 22.

L'invention réside dans le fait que la face d'entrée du détecteur 22 est masquée par une bande 23 qui absorbe le rayonnement X sauf en des zones dont les dimensions variables sont prévues pour s'adapter aux différentes formes de seins. La bande 23 est entraînée par un moteur 24 et s'arrête sous la commande du praticien pour que la zone appropriée soit en correspondance avec la face d'entrée du détecteur 22.

## FIG. 2

L'invention concerne les systèmes de radiologie, notamment les mammographes et plus particulièrement dans de tels systèmes, un dispositif qui permet de contrôler la dose de radiation reçue par la personne en cours d'observation ainsi que la durée de la pose de manière à obtenir une image avec le contraste optimal pour une dose de radiation minimale.

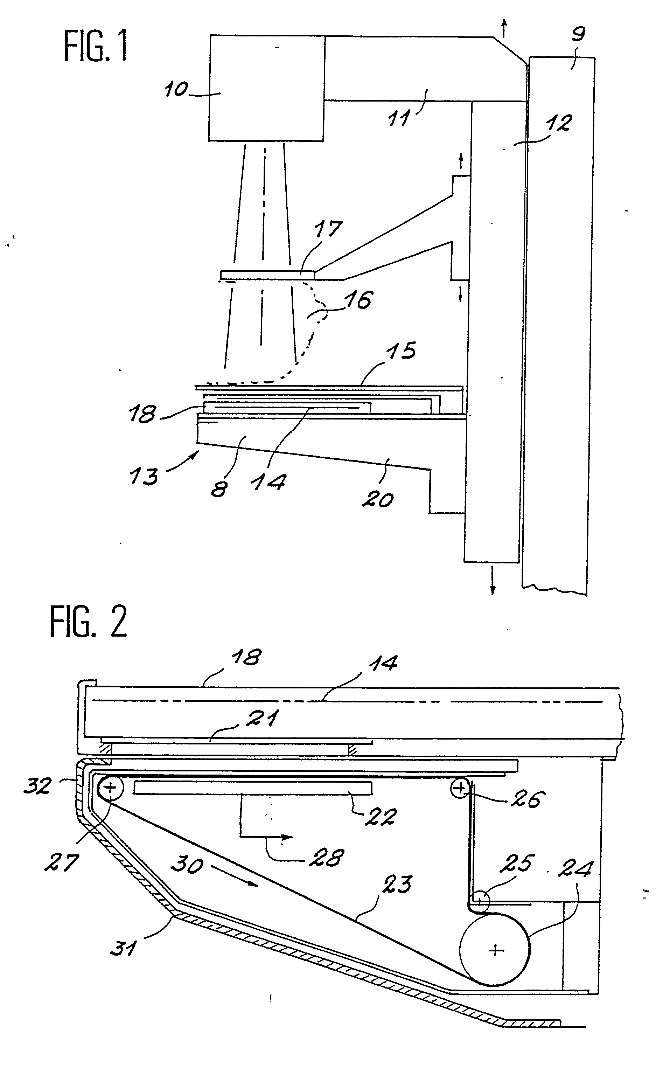

Les systèmes de radiologie du type mammographe comprennent, comme le montre la figure 1 une source 10 de rayonnement X portée par une potence 11 disposée au sommet d'une plaque verticale 12. Cette dernière comporte un ensemble 13 sur lequel repose le sein 16 à examiner par l'intermédiaire d'une tablette 15. Une pelote 17 transparente au rayonnement X et mobile verticalement sur la plaque 12, sert à comprimer le sein.

Pour s'adapter à la taille de la patiente, la plaque 12 est montée sur une colonne verticale 9 reposant sur le sol et se déplace verticalement sur ladite colonne grâce à un dispositif mécanique approprié.

L'ensemble 13 présente sur sa partie supérieure et sous la tablette 15, un tunnel dans lequel vient se loger une cassette 18. Cette cassette est constituée d'une boîte noire renfermant un film 14 sensible au rayonnement X direct ou à un rayonnement photonique émis par un écran (non représenté) recevant le rayonnement X. C'est sur ce film 14 que se forme l'image latente du sein après une durée de pose appropriée; le développement du film donne un cliché radiographique.

Pour que le cliché présente un intérêt en vue d'un diagnostic, tous les points qui constituent l'image de l'objet examiné doivent présenter entre eux un contraste suffisant; en particulier, le noircissement du film doit être correct et "normalisé" pour un domaine très large d'opacité de l'objet. A cet effet, le noircissement peut être contrôlé par un dispositif de contrôle de radiation qui est disposé sous la cassette 18 dans la partie inférieure 8 de l'ensemble 13. Ce dispositif de contrôle, également appelé exposeur, est constitué d'un détecteur de rayonnement X qui délivre un signal électrique proportionnel au débit de la dose de rayonnement X qui passe à travers le film sensible. Ce signal électrique, qui traduit l'intensité du rayonnement X, est intégré pendant le temps de pose et le signal résultant de cette intégration est comparé à chaque instant à un signal de seuil pré-déterminé qui est fonction des caractéristiques du film sensible. Dès que le signal intégré atteint ce seuil, le signal indiquant l'égalité commande l'arrêt de la source , ce qui termine la pose.

Un des avantages de ce dispositif de contrôle de radiation est de permettre, pour une large plage de variation des débits de dose de rayonnement X qui conduisent à des différences de pose, d'une part, d'obtenir une exposition du film sensible correspondant à un contraste optimal et, d'autre part, de mieux contrôler la dose moyenne reçue par le patient, dose qui constitue un facteur important pour l'évaluation du risque carcinogène.

Dans un dispositif de contrôle de radiation, il est important que le détecteur ne reçoive que le rayonnement ayant traversé le sein car la réception d'un rayonnement X non atténué fausserait la mesure. Aussi, la surface réceptrice d'un tel détecteur est limitée par la taille du plus petit sein à examiner. Une telle limitation restreint considérablement les avantages que l'on peut tirer de ce dispositif et constitue un facteur d'erreur dans certaines circonstances car la zone de l'objet correspondant à la taille du détecteur peut être différente de celle que l'on examine. En effet, la position du détecteur est en général fixe alors que la zone à examiner peut avoir une position variable par rapport à celle du détecteur et il n'y a donc pas le recouvrement souhaité en vue d'une mesure optimale.

Cette limitation est encore plus marquée en mammographie car il y a une grande disparité entre les individus qui sont observés et, pour un même individu, une disparité selon le moment auquel est effectué l'examen par rapport au cycle hormonal. Dans la première catégorie, il y a les différences anatomiques comme la taille du sein ou la composition locale des tissus. Dans la deuxième catégorie, il y a la composition et la distribution des tissus en fonction du cycle hormonal, de l'âge, du poids, du développement somatique. En outre, il y a la densité et la distribution des structures à visualiser, qu'elles soient pathologiques ou non, qu'elles soient massives ou qu'il s'agisse de micro-calcifications dont les positions ne sont pas connues du praticien.

En résumé, avec un détecteur de position fixe et de petites dimensions, le signal de mesure n'est pas représentatif du sein dans son ensemble et peut conduire à des clichés sous-exposés dans le cas où le détecteur est sous une partie adipeuse du sein ou surexposés dans le cas où le détecteur est sous une partie fibreuse ou sous une opacité pathologique.

Par suite des insuffisances évoquées ci-dessus, les clichés obtenus seront difficilement exploitables par le praticien pour effectuer avec un grand degré de certitude un diagnostic ou un dépistage préventif. Il sera donc amené à refaire l'examen de sorte que les avantages de l'utilisation d'un détecteur, savoir la rapidité, le meilleur contraste, la réduction de la dose de rayonnement et du flou cinématique sont compromis.

Ces inconvénients sont en partie atténués dans les systèmes où tout l'ensemble détecteur peut être déplacé dans son plan au-dessous du sein. Cependant, il existe une limite à la plus grande dimension possible du détecteur et, de ce fait, ce dernier est mal optimisé aux différentes dimensions de sein rencontrées. Le signal résultant de l'intégration est, dans ce cas, une approximation du signal optimum : c'est donc l'expérience qui doit guider le praticien dans le choix de la position du détecteur.

Par ailleurs, il n'est guère possible de prédire où seront les opacités sur le cliché, d'où la difficulté de choisir la position de la cellule au premier cliché.

Le but de la présente invention est donc de réaliser un dispositif de contrôle de radiation qui s'adapte aux différentes tailles de l'objet à radiographier.

A cet effet, l'invention propose, d'une part, d'agrandir les dimensions du détecteur jusqu'à atteindre éventuellement celles du film sensible et, d'autre part, d'utiliser des masques de différentes dimensions qui sont placés entre la cassette et le détecteur ou entre le film sensible au rayonnement X et le détecteur.

L'invention se rapporte à un dispositif de contrôle de radiation dans un système de radiologie qui comprend au moins une source de rayonnement X émettant un faisceau qui irradie un objet à observer, un film sensible placé sous l'objet sur lequel se forme l'image latente dudit objet et un détecteur du rayonnement X ayant traversé l'objet à observer et le film sensible, ledit détecteur fournissant un signal électrique qui sert à contrôler le temps de pose de l'objet, caractérisé en ce qu'il comprend en outre au moins un masque à opacité appropriée à l'énergie du rayonnement X qui est placé entre le film sensible et le détecteur, ledit masque présentant au moins une zone qui n'absorbe pas le rayonnement X, la forme et la surface de la zone non-absorbante étant adaptées aux dimensions de l'objet à observer et/ou au type d'examen qui est effectué.

Dans une forme de réalisation simplifiée de l'invention, le masque est supporté par un cadre qui est disposé de manière amovible devant le détecteur.

Dans une forme de réalisation plus élaborée, le masque est supporté par une bande qui est associée à un mécanisme de déplacement de ladite bande de manière à placer ladite zone non-absorbante du rayonnement X à une position optimale par rapport à l'objet à observer.

En outre, cette bande comporte plusieurs zones non absorbantes du rayonnement X qui sont disposées de préférence successivement dans le sens de déplacement de la bande et dont les dimensions et la forme sont différentes d'une zone à la suivante de manière à s'adapter aux dimensions et à la forme de l'objet ainsi qu'au type d'examen effectué.

Le détecteur de rayonnement X peut être du type comportant une chambre d'ionisation ou du type à semi-conducteur.

D'autres caractéristiques et avantages de la présente invention apparaitront à la lecture de la description suivante d'exemples particuliers de réalisation, ladite description étant faite en relation avec les dessins joints dans lesquels :

- la figure 1 est une vue en coupe verticale d'un exemple particulier de réalisation d'un mammographe dans un plan passant par la source du rayonnement X,

- la figure 2 est une vue en coupe verticale d'un exemple particulier de réalisation d'un dispositif automatique de changement de masque selon l'invention,

- la figure 3 est une vue déployée de la bande montrant les différentes zones non-absorbantes au rayonnement X,

- la figure 4 est une vue en perspective d'un dispositif à tiroir permettant de changer manuellement les masques du détecteur en fonction des dimensions anatomiques du sein, et

- les figures 5a et 5b, montrent les formes et les dimensions respectives de deux masques à positionnement manuel.

L'invention sera décrite dans son application à un mammographe mais elle peut être mise en oeuvre dans d'autres systèmes de radiologie où il est fait usage d'un contrôleur de radiation du type qui convertit un rayonnement X en un signal électrique. Afin de mieux adapter le contrôle de radiation à la zone à examiner quelles que soient les dimensions du sein, l'invention propose de modifier la surface de la zone active du détecteur de rayonnement en plaçant au-dessus de ce dernier un masque absorbant le rayonnement X qui présente des fenêtres non-absorbantes audit rayonnement dont les dimensions et la forme varient en fonction de celles du sein à examiner.

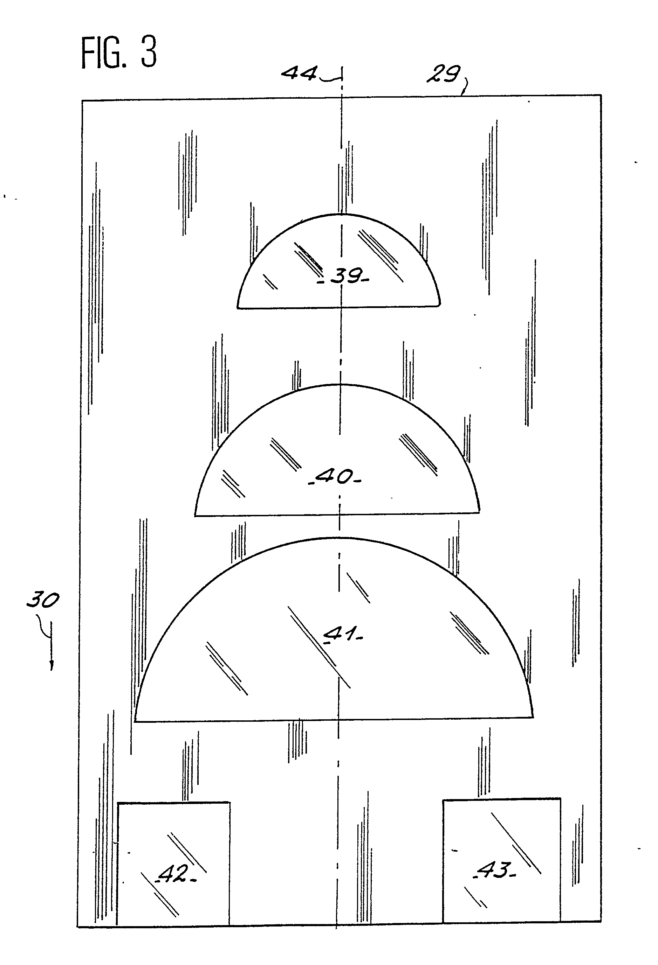

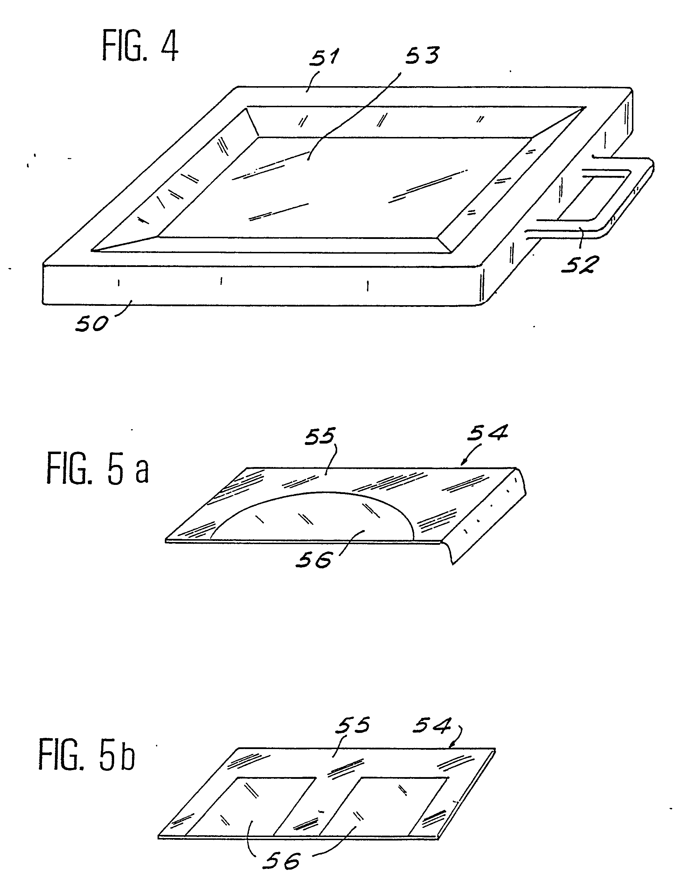

Pour réaliser une telle interposition d'un masque, l'invention propose un dispositif automatique (figures 2 et 3) et un dispositif manuel (figures 4 et 5).

Le dispositif automatique est disposé dans la partie inférieure 8 de l'ensemble 13 sous la cassette 18. Cette dernière comporte de manière connue, un film sensible 20 à l'intérieur d'une pochette ou d'une boîte noire que constitue la cassette et une zone 21 non-absorbante du rayonnement X qui est située dans la paroi inférieure de la cassette 18 et à proximité du bord extérieur 32 de la partie la plus près du patient. Le détecteur de radiation 22 est constitué, par exemple, d'une chambre d'ionisation ou d'un dispositif à semi-conducteur qui fournit un signal électrique sur un conducteur 28 lorsqu'il reçoit un rayonnement X traversant la zone 21. Le détecteur 22 est fixé sous la fenêtre 21 à un châssis 31 constitué essentiellement par la partie inférieure 8 de l'ensemble 13. Sur le dessus du détecteur 22 est disposée une bande 23 qui est supportée et entraînée en mouvement suivant la flèche 30 par un moteur d'entraînement 24 et des galets de support 25,26 et 27.

La bande 29 est refermée sur elle-même et absorbe le rayonnement X sur toute sa surface à l'exception de zones 39,40,41,42 et 43 (figure 3). Les zones 39,40 et 41 ont une forme générale de demi-cercle dont le diamètre varie d'une zone à la suivante, la zone 39 de plus petit diamètre étant prévu par exemple pour l'examen d'un sein de petites dimensions, la zone 40 pour l'examen d'un sein de dimensions moyennes et la zone 41 pour l'examen d'un sein de grandes dimensions. Enfin, les zones 42 et 43, qui sont de forme rectangulaire et disposées symétriquement de part et d'autre de l'axe de symétrie longitudinale 44 de la bande, sont utilisées lors d'un examen stéréotaxique pour reproduire deux vues sur le même film.

Selon la longueur de la bande 29, il est possible de créer un nombre de fenêtres, telles que 39,40 et 41, supérieur à trois afin de mieux couvrir la disparité des dimensions des seins. En outre, ces zones peuvent avoir des formes autres que celle d'un demi-cercle. Bien entendu, les dimensions maximales de la fenêtre 41 ou du rectangle englobant les fenêtres 42 et 43 sont celles de la face d'entrée du détecteur 22.

Au cours d'un examen, le choix de la fenêtre qui est adaptée au sein observé est effectué par le praticien et le circuit de commande électrique du moteur d'entraînement 24 est prévu pour mettre en place la fenêtre choisie devant la face d'entrée du détecteur 22, la base du demi-cercle étant située vers le bord extérieur 32 de l'ensemble 13 le plus près possible du patient.

Les figures 4 et 5 donnent un exemple de réalisation d'un dispositif manuel. Il comprend un tiroir 50 (figure 4) qui est disposé entre la cassette 18 ou le film en pochette et le détecteur 22 (figure 2). De préférence, ce tiroir est placé parallèlement à la paroi inférieure de la cassette 18, sous la zone 21, ou directement sous la pochette qui contient le film sensible au rayonnement X. Le tiroir 50 consiste en un cadre 51 muni d'une poignée 52 de manipulation. La zone centrale 53 n'absorbe pas le rayonnement X et son pourtour sert de support à un masque 54 (figure 5) constitué d'une zone extérieure 55 opaque au rayonnement X et une zone centrale 56 comportant une fenêtre transparente au rayonnement X. Il est prévu d'utiliser plusieurs masques dont les dimensions de chaque fenêtre varient d'un masque à l'autre, chaque fenêtre correspondant par exemple à une de la bande représentée sur la figure 3.

Dans le cas des figures 4 et 5, le tiroir 50 est prévu pour s'emboîter dans l'ensemble 13, de préférence par un des deux côtés latéraux parallèles au plan de la figure 1. Il est possible aussi d'emboîter le tiroir par le bord extérieur 32 mais dans ce cas il est nécessaire de le faire avant le positionnement de la patiente.

Dans le cas du dispositif automatique des figures 2 et 3, le sens du déplacement de la bande 29 a été prévu suivant le sens de la flèche 30. Il est clair que l'invention peut également être mise en oeuvre à l'aide d'une bande qui se déplacerait suivant un sens perpendiculaire au plan de la figure 1. Dans ce cas les fenêtres 39 à 43 devront être tournées de 90° de manière que la base du demi-cercle soit parallèle au sens de déplacement.

La bande 29 peut, par exemple, être réalisée à l'aide d'un matériau souple qui est recouvert d'un matériau à opacité appropriée à la plage du rayonnement X utilisé sauf à l'endoit des zones non-absorbantes 39 à 43. Ce matériau opaque au rayonnement X doit être suffisamment souple pour ne pas trop diminuer la souplesse de la bande 29 et ne pas présenter de fissures, notamment, après une longue utilisation car ces fissures fausseraient les mesures du détecteur 22.

## Revendications

1. Dispositif de contrôle de radiation dans un système de radiologie qui comprend au moins une source (10) de rayonnement X émettant un faisceau qui irradie un objet (16) à observer, un film sensible (14) placé sous ledit objet (16) sur lequel se forme l'image latente de l'objet et un détecteur (22) du rayonnement X ayant traversé l'objet (16) à observer du type comportant une chambre d'ionisation (22) ladite chambre fournissant un signal électrique qui sert à contrôler le temps de pose de l'objet, caractérisé en ce qu'il comprend en outre un masque (23,54) opaque au rayonnement X qui est placé au-dessus de la chambre d'ionisation (22), ledit masque présentant au moins une zone transparente (39,43,56) au rayonnement X, la forme et la surface de la zone transparente étant adaptées aux dimensions de l'objet à observer et/ou au type d'examen qui est effectué.

2. Dispositif de contrôle selon la revendication 1, caractérisé en ce que le masque (54) est supporté par un cadre (51) qui est disposé de manière amovible au-dessus de la face d'entrée de la chambre d'ionisation (22).

3. Dispositif de contrôle selon la revendication 1, caractérisé en ce que le masque est supporté par une bande (29) qui est associée à un mécanisme de déplacement (24 à 27) de ladite bande de manière à placer ladite zone transparente à une position optimale par rapport à l'objet.

4. Dispositif de contrôle selon la revendication 3, caractérisé en ce que la bande comporte plusieurs zones transparentes (39 à 43) au rayonnement X qui sont disposées successivement dans le sens de déplacement de la bande (23) et dont les dimensions et la forme sont différentes d'une zone à la suivante de manière à s'adapter aux dimensions et à la forme de l'objet ainsi qu'au type d'examen qui est effectué.

FIG.1

FIG. 2

FIG. 3

FIG. 4

51   53

52

50

FIG. 5 a

55   54

56

FIG. 5 b

55   54

56

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-3 824 397  (BAUER et al.) <br> * Colonne 1, ligne 50 - colonne 2, ligne 3; colonne 2, lignes 34-39; figures 5,6 * <br> --- | 1-4 | A 61 B   6/00 <br> G 01 T   1/29 |
| A | EP-A-0 158 838  (FUJI PHOTO FILM CO., LTD) <br> * Abrégé; page 24, ligne 21 - page 25, ligne 11; page 26, ligne 23 - page 27, ligne 3; figures 1-9 * <br> --- | 1-4 | |
| A | RADIOLOGY, vol. 125, 1978, pages 517-523, US; E.P. MUNTZ et al.: "Preliminary studies using electron radiography for mammography" <br> * Page 517 - page 518, colonne 1; figures 1,3 * <br> ----- | 1-4 | |

| | | | **DOMAINES TECHNIQUES RECHERCHES (Int. Cl.5)** |
|---|---|---|---|
| | | | G 01 T <br> A 61 B |

Le présent rapport a été établi pour toutes les revendications

| Lieu de la recherche | Date d'achèvement de la recherche | Examinateur |
|---|---|---|
| LA HAYE | 09-08-1990 | DATTA S. |